(19) Europäisches Patentamt
European Patent Office
Office européen des brevets

(11) Publication number: **0 205 232**
**A1**

(12) # EUROPEAN PATENT APPLICATION

(21) Application number: 86302454.3

(22) Date of filing: 02.04.86

(51) Int. Cl.[4]: G 01 N 21/64
G 01 N 21/77

(30) Priority: 08.04.85 US 720749
08.04.85 US 720750

(43) Date of publication of application:
17.12.86 Bulletin 86/51

(84) Designated Contracting States:
AT BE CH DE FR GB IT LI LU NL SE

(71) Applicant: KELSIUS, INC.
957P Industrial Road
San Carlos California 94070(US)

(72) Inventor: Klainer, Stanley M.
20 Belinda Court
San Ramon California 94563(US)

(72) Inventor: Walt, David R.
12 Milk Street
Lexington Massachusetts 02173(US)

(74) Representative: Allard, Susan Joyce et al,
BOULT, WADE & TENNANT 27 Furnival street
London EC4A 1PQ(GB)

(54) **Amplification of signals from optical fibers.**

(57) An article comprising a solid substrate, in particular an optical fiber, to the surface of which are attached a plurality of polymer molecules, the polymer molecules embodying a multiplicity of chemically, biochemically or biologically active species.

A method of preparing an optical fiber which is useful for sensing a chemical or physiological parameter of a body fluid or tissue (a) comprises providing an optical fiber having a distal end, and (b) treating the distal end of the fiber to attach to it a polymer embodying a multiplicity of molecular species which are capable of sensing the desired parameter.

EP 0 205 232 A1

AMPLIFICATION OF SIGNALS FROM OPTICAL FIBERS

This invention relates to the use of optical fibers for chemical and biochemical purposes such as the measurement of chemistries in physiological tissues and fluids.

The use of optical fibers for such purposes is known and is described, for example, in Hirschfeld, U.S. Patent Application Serial No. 194,684 filed October 6, 1980, entitled "Remote Multi-Position Information Gathering System and Method." Such technique is also described in a paper entitled "Novel Optical Fiber Techniques For Medical Application" by F.P. Milanovich, T.B. Hirschfeld, F.T. Wang, S.M. Klainer, and D. Walt, published in Proceedings of the SPIE 28th Annual International Technical Symposium on Optics and Electrooptics, Volume 494.

Briefly summarizing this technique, a sensor consisting typically of but not limited to a fluorescent dye is attached to the distal end of an optical fiber of suitable diameter for in vivo application. Light from an appropriate source is used to illuminate the distal end of the optical fiber, such light having a suitable wave length, for example 488 nanometers. The light propagates along the fiber and a portion of this light is reflected at the distal end at the fiber-liquid interface and returns along the fiber. A second portion of the light of this wavelength is absorbed by the fluorescent dye and light is emitted in a band centered usually but not always at a

longer wavelength. The intensity of this band is dependent upon the interaction of the dye and the measured substance in the body fluid. This fluorescent light is also propagated along the return path of the fiber and by suitable optical equipment such as that described in the patent and other literature and in the Hirschfeld application and the paper above identified, the ratio of the wavelengths is determined and from this the parameter of interest is measured.

Fluorescent molecules have been attached to the distal end of an optical fiber or to a separate piece of glass such as, for example, a glass bead by a technique which is used in the immobilization of enzymes. See, for example, Methods in Enzymology, vol. XLIV, Immobilized Enzymes, edited by Klaus Mosbach, Academic Press, 1976, pages 134-148.

In this procedure a glass surface, for example, the distal end of an optical fiber or a glass bead which is subsequently welded to the fiber, is preferably treated so that it is porous and it is reacted with a suitable linking agent such as 3-aminopropyl triethoxy silane. A series of reactions is carried out culminating in the attachment by covalent bonds of a biologically and/or chemically active molecule, e.g., a fluorescent species, to the surface of the glass. These reactions may be represented as follows:

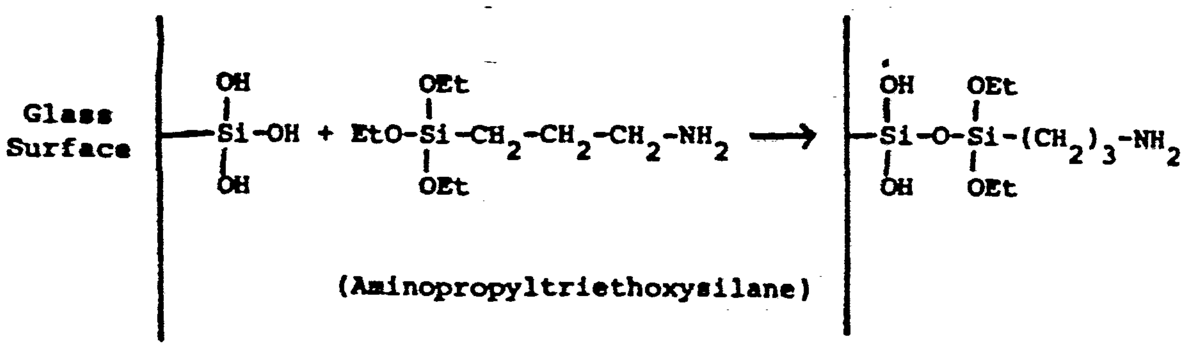

(Aminopropyltriethoxysilane)

Representing the silanized glass (referred to also hereinafter as aminoalkyl glass or fiber) as

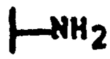

The next step is realized by reacting fluorescein isothiocyanate dissolved in an aprotic solvent with the aminoalkyl fiber

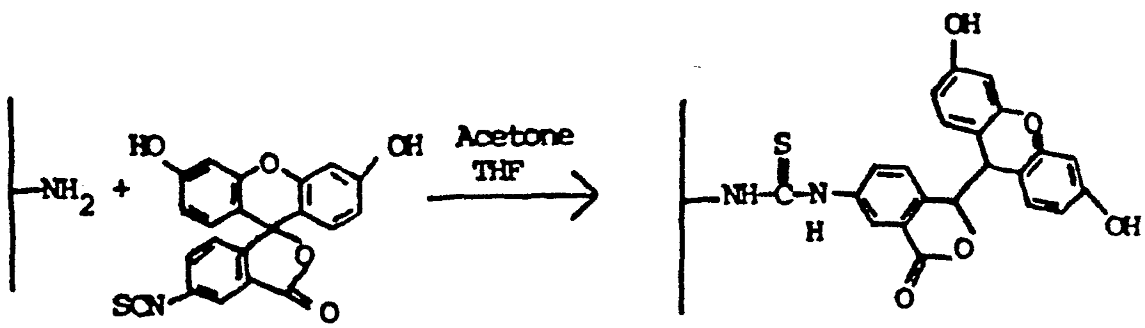


and performing the remaining conventional washing and purification steps.

A dye immobilized on glass in this manner is pH sensitive in the physiological range and is relatively stable. However, to achieve a satisfactory degree of response it is necessary to employ porous glass, that is a special alkali borosilicate glass which has been heat treated to separate the glass into two phases. The phase which is rich in boric acid and alkali is leached out using acid to leave a porous high silica structure. The porous glass is then silanized and is treated with the desired fluorescent species. The resulting treated glass must be attached to the distal end of an optical fiber. The effect of using porous glass is that a greater surface area is available for silanizing and many more molecules of the

fluorescent species can therefore be attached. That is to say, the signal emission of fluorescent light is of greater intensity than if the end of the fiber itself were silanized and treated with a fluorescent species.

However, this technique is difficult to carry out. The most practical way to accomplish the intended result is to provide a bead of porous glass, silanize it, react it with the fluorescent species and then attach the glass bead to the distal end of an optical fiber. This attachment is difficult to effect due to the small size involved and the ease with which the pores in the porous glass are occluded.

It is an object of the present invention to provide a better way of enhancing or amplifying the signal from fluorescent or other chemically/biologically active species attached to glass and to glassy/optically transparent organic substitutes.

It is a particular object of the invention to provide a means of amplifying such signals which avoids the need to employ porous glass.

The above and other objects of the invention will be apparent from the ensuing description and the appended claims.

In accordance with the present invention glass (or a glass substitute) which is non-porous is treated to attach suitable linking groups, e.g., by silanizing the glass, and a polymer is attached covalently to the linking groups as, for example, by reacting the linking groups with a monomeric species and then adding further monomer species and forming a polymer in situ or by attaching a preformed polymer to the silanized glass. The monomeric species or the polymer may have pendant fluorescent groups, or the

monomeric reactant may be a mixture of fluorescent and non-fluorescent species. By this means, polymer molecules having a multiplicity of pendant fluorescent groups, are covalently attached to the glass. The multiplication of fluorescent (or other chemically or biologically active) groups greatly amplifies the desired signal.

Alternatively the polymer covalently attached to the glass may itself be non-fluorescent but may serve to entrap fluorescent molecules.

In yet another embodiment the glass is treated so that basic groups such as amino groups are attached covalently to the glass and a polymer having pendant fluorescent groups and also acidic groups, e.g., carboxy groups, is reacted with the glass to form salts in which the cations are formed by the basic groups of the glass and the anions are formed by the acidic groups of the polymer, e.g.

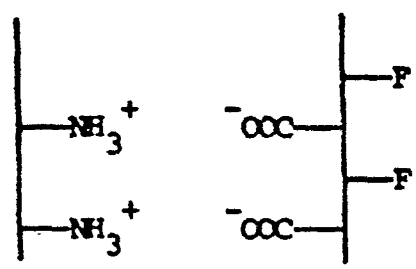

A B

where A represents the treated glass, B represents the polymer and F represents fluorescent groups.

In another embodiment a fluorescent polymer is held on a treated glass surface by adsorption.

In general, therefore, the product of the invention is an optical fiber having attached to its distal end one or more (usually more) polymer molecules which serve to attach chemically or biologically active entities to the fiber. By this means the desired effect, e.g.

fluorescence, is greatly amplified and the need to employ porous glass is avoided.

The following specific examples will serve further to illustrate the invention.

**Example 1: Preparation of Acid Glass**

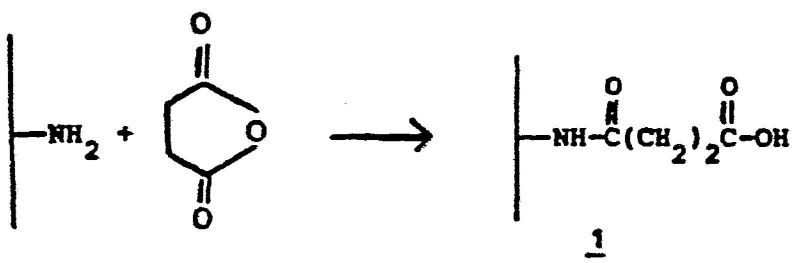

1

In 1 and generally hereinafter, the group --NH-- is derived from the primary amino group of the aminoalkyl glass.

The acid glass 1 was prepared as follows: Succinic anhydride (3.0g) was dissolved in anhydrous tetrahydrofuran (THF)(100ml). Silanized glass, i.e. aminoalkyl glass, (1g) was added and the solution was refluxed for four hours. The glass was then washed with 30ml of acetone and dried in vacuo at 25°C for thirty minutes.

**Example 2 - Preparation of Acid Chloride Glass**

The acid glass of Example 1 was treated with $SOCl_2$ as follows: A 10% solution of thionyl chloride in dry chloroform was prepared. 0.5g of acid glass was added to 20ml of the thionyl chloride solution and was refluxed for four hours. The glass was then rinsed with chloroform and dried under vacuum for thirty minutes.

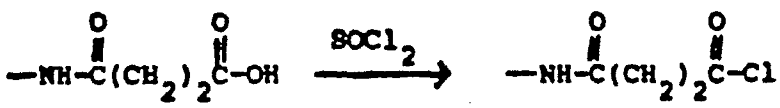


1 2

**Example 3 - Preparation of N-Hydroxy Succinic (NHS) Glass**

The acid chloride glass of Example 2 was reacted with N-hydroxy-succinimide as follows:

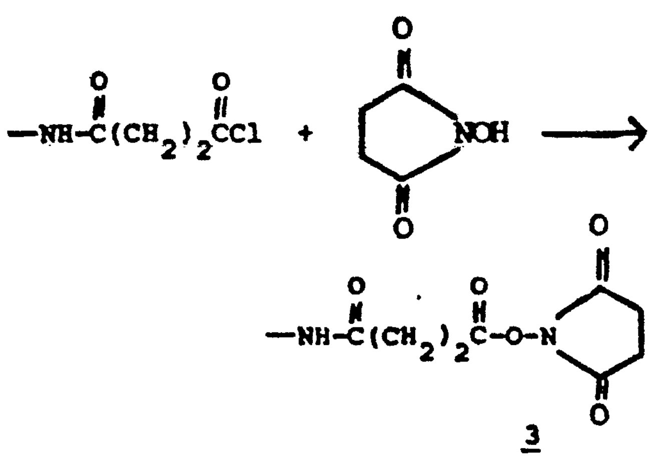

3

The reaction was carried out as follows: 3.8g of N-hydroxysuccinimide and 4.45 ml of triethylamine were added to 50 ml of anhydrous stirred chloroform. This solution was then cooled to 0°C and with additional cooling so as to maintain a constant temperature of 0°C. Acid chloride glass was added and the suspension was stirred for an additional forty minutes. The glass was filtered, the beads washed with chloroform and dried in vacuo at 25°C for thirty minutes.

The purpose of treating the acid chloride glass with N-hydroxy succinimide was to protect the treated glass from hydrolysis in subsequent treatment.

**Example 4 - Treatment of NHS Glass with Triethylenetetramine (TET)**

The NHS glass from Example 3 is added with stirring to a mixture of 50 ml of anhydrous chloroform and 8 ml of triethylenetetramine. The glass mixture is stirred for one hour, the beads are then filtered, washed with chloroform and dried in vacuo at 25°C for thirty minutes. The reaction may be represented as follows:

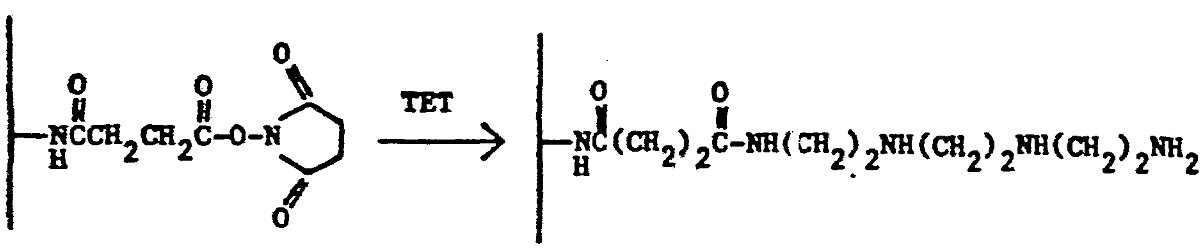


4

The treated glass 4 has numerous nitrogen functionalities to which fluorescent groups may be attached.

**Example 5 - Glutaraldehyde (glut-ald) Glass**

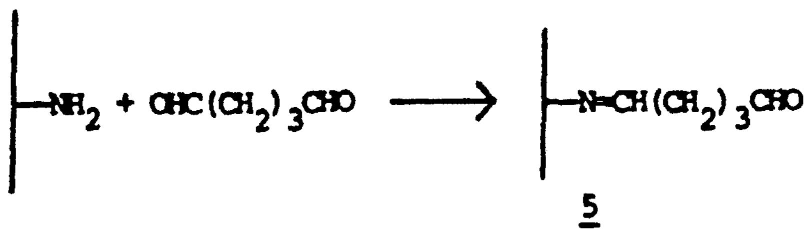

5

This was prepared as follows: A solution of 25% glutaraldehyde was diluted to 2.5% in o.1M $Na_3PO_4$ of pH7 to make 50 ml. The aminoalkyl glass was immersed for one hour and then rinsed with deionized water.

**Example 6 - Glutarylchloride (glut-Cl) Glass**

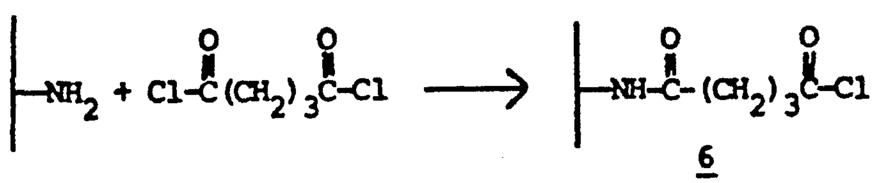

6

2.5 ml of glutaryl dichloride was diluted to 10 ml in anhydrous THF. Aminoalkyl glass (400mg) was immersed for one hour in this solution and was then rinsed with dry acetone.

**Example 7 - Attachment of a Fluorescent Polymer to Aminoalkyl Glass**

(a) Preparation of N-substituted acrylamide derivative of fluorescein

200 mg of fluorescein amine

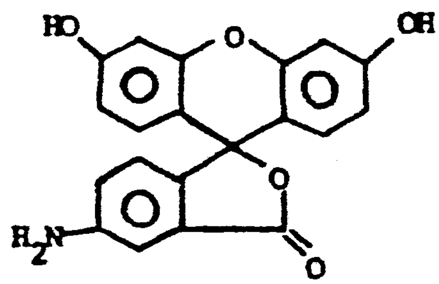

7

were dissolved in a mixture of 1.0 ml anhydrous dimethyl formamide and 25 ml anhydrous chloroform followed by adding 0.56 ml of acryoyl chloride with stirring. The reaction mixture was stirred and cooled throughout the course of the reaction to maintain a temperature of 25°C. After sixty minutes the pH of the reaction mixture was about 3. The solvent was stripped off in vacuo leaving an amber oil. The product had the formula 8

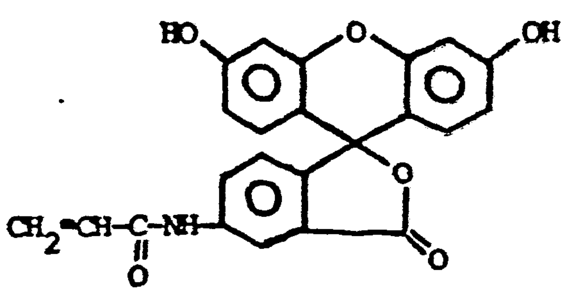

8.

Compound 8 is a new and useful compound.

(b) Copolymerization of Acrylamide and 8 with Aminoalkyl Glass

400 ml of 8 were dissolved in 30 ml tetrahydrofuran together with 1 gram acrylamide and 10 mg of 2,2'- azobisisobutyro nitrile (AIBN) as catalyst. Aminoalkyl glass in the form of a fiber tip was activated in 1 ml acryloyl chloride for one hour and then immersed in the solution and heated at 50°C for 20 hours. A copolymer of 8 and acrylamide linked to the aminoalkyl glass resulted which is believed to have the structure 9:

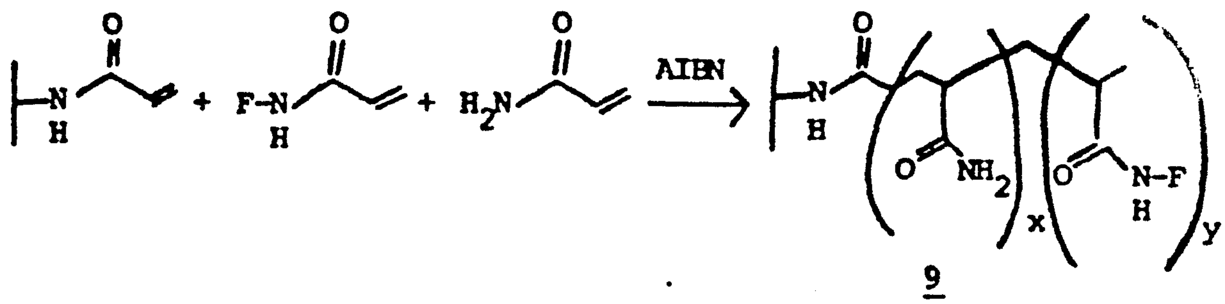


9

In 9 x and y indicate the numbers of the respective mer units:

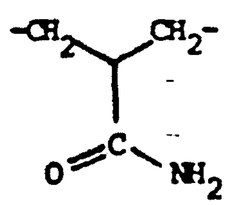

and

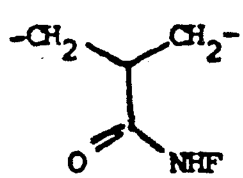

The mer units in all likelihood occur in some more or less random order. The pendant group F is derived from 8; i.e. it is the group 8a

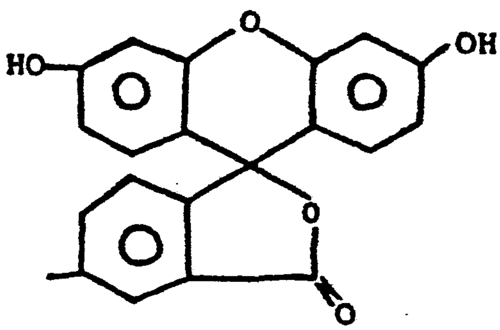

8a

(c) Testing of Fiber Tip 9

The fiber tip 9 was tested by immersing it in a buffer solution at pH 6.8 and then at 7.8. The ratio of the intensity of light resulting from fluorescence and reflected light at pH 6.8 was 0.81 and at pH 7.8 it was 1.1.

(d) Comparison with a Porous Glass Bead to which FITC is Directly Attached

A porous glass bead, OD = 177 μ, average pore size = 500 angstrom units was glued to the end of an optical fiber. It was then treated with aminopropyl triethoxy silane to activate the porous glass. Due to the porous nature of the glass, many aminoalkyl groups were presumably attached. This was done by treating the porous glass with a 10% aqueous solution of aminopropyl triethoxy silane at pH 3.45 for 60 minutes. The fiber was then rinsed in distilled water and air dried at 90°C.

Fluorescein isothiocyanate was dissolved in acetone and the tip of the fiber described above was immersed in the solution for 60 minutes at room temperature. The fiber was rinsed with acetone. This fiber was tested as in Example 7(c) above. The ratio of intensity of light resulting from absorption to reflected light at pH 6.8 was 0.54 and at pH 7.8 it was 1.29.

In Example 7(c) the glass to which the fluorescent species was attached was non-porous. It was treated to append aminoalkyl groups but eliminated the tedious step of attaching a porous bead.

Example 8 - Glass Fiber Treated with FITC

By way of further comparison, aminoalkyl glass fiber as in Example 7(b) was treated as follows: The fiber was soaked in a solution containing 5 mg of fluorescein isothiocyanate (FITC) in 100 ml of acetone for one hour and then in deionized water for one hour. No observable fluorescent pH effect could be measured using the procedure of Example 7(c).

Example 9 - Glass Fiber Treated with High Molecular Weight Polylysine and FITC

A glutaraldehyde treated aminopropyl glass fiber (prepared as in Example 5) was placed in a solution of 4 mg of 540,000 average molecular weight polylysine in 4 ml of

deionized water and allowed to soak for one hour at 23°C. The fiber was then allowed to stand in a deionized water wash for one hour and then treated with a solution of 5 mg FITC/100 ml acetone for one hour. The fiber was then rewashed in deionized water for one hour and its pH dependent fluorescence measured. A pH 6.8, the ratio of fluorescence to reflected light, was 0.11 and at pH 7.4 it was 0.21.

**Example 10 - Glass Fiber Treated with Low Molecular Weight Polylysine and FITC**

A glutaraldehyde treated aminopropyl glass fiber (prepared as in Example 5) was placed in a solution of 4 mg of 4,000 average molecular weight polylysine in deionized water and allowed to soak for one hour at 23°C. The fiber was then placed in a deionized water wash for one hour and immediately treated with a solution of 5 mg FITC/100 ml acetone for one hour. The fiber was then rewashed in deionized water for one hour and its pH dependent fluorescence measured. The ratio of fluorescent to reflected light at pH 6.8 was 0.35 and at pH 7.4 it was 0.52.

From the results of Examples 9 and 10 it is concluded that polylysine is attached covalently to the aminopropyl glass and that FITC is attached covalently as pendant groups to the polylysine chains.

**Example 11 - Entrapment of a Fluorescent Species in a Polymer Covalently Attached to Glass**

An aminopropyl glass fiber is treated with acryloyl chloride, by immersion in 1.0 ml of neat acryloyl chloride for 60 minutes and is then rinsed by soaking in dry acetone. 2.0 g of acrylamide and 0.01 g of fluorescein are dissolved in 50 ml THF and a catalytic amount of AIBN is added. The treated fiber described above is immersed in

the solution through a septum. The reaction flask is flushed with $N_2$ and placed in a water bath at 50°C for three to four hours, until a polymer is formed. The fibers are rinsed in acetone and water. A polymer is formed as in Example 7 but without fluorescent moieties. The fluorescein is physically entrapped in the non-fluorescent polymer.

If instead of acrylamide a monomeric species is used which is capable of cross linking, e.g., methylene bisacrylamide,

$$CH_2{=}CH{-}CO{-}NH{-}CH_2{-}NH{-}CO{-}CH{=}CH_2$$

a cross linked polymer will be formed on the glass which is more effective in entrapping the fluorescent molecules.

**Example 12 - Electrostatically Bound Fluorescent Polymer**

A fluorescent polymer is prepared having pendant fluorescent groups and pendant carboxy groups and it is applied to activated glass having basic groups, resulting in the following:

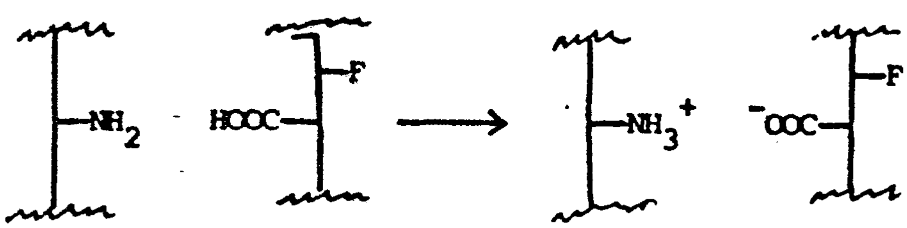

C D E

where C represents one of a multiplicity of activated groups on glass, D represents a repeating unit in the polymer described above (wherein F represents a pendant fluorescent group) and E represents the resulting salt.

This may be accomplished by reacting aminopropyl glass <u>C</u> with a fluorescently labeled polymer <u>D</u>. The resulting ionic salt bridges are then stabilized by drying the product <u>in vacuo</u>.

**Example 13 - Adsorption of a Fluorescent Polymer on an Aminoalkyl Glass Surface**

A polymer having pendant fluorescent groups is prepared, e.g., a copolymer of acrylamide and the acryolyl derivative <u>8</u> of fluorescein prepared, as described in Example 7(b) but in the absence of aminopropyl glass.

An aminopropyl glass fiber tip is immersed in a 0.01 molar aqueous solution of this copolymer for two days. The fiber is then rinsed with chloroform for three days to remove the non-adsorbed polymer and is dried in vacuo at 25°C for one hour.

**Example 14 - Immunoassay Embodiment**

In the enzyme linked immunoassay (ELISA) technique an enzyme is immobilized on the surface of a test tube or cuvette. See, for example, Biochemical Applications of Immobilized Enzymes and Proteins, Vol. 2, T. M. S. Chang, Plenum Press, 1977, Chapters 30-33. This technique is improved by the amplification technique of the present invention, e.g., as follows:

An acetaminophen-specific antibody is treated with a one-hundred fold molecular excess of acryloyl chloride, and taken into solution with deionized water to which a two fold quantity of acrylamide has been added together with a suitable amount of AIBN. An aminopropyl glass optical fiber is treated with an excess of acryloyl chloride, washed, added to the acrylated antibody/AIBN solution and warmed at a sufficient temperature to promote graft

polymerization of the antibody derivative to the silica surface without denaturing the protein.

Next an acetaminophen-alkaline phosphatase conjugate is incubated with the immobilized antibody, filtered, washed and the unknown quantity of acetaminophen-analyte added. The residual bound enzyme activity is then measured and the quantity of acetaminophen calculated.

Glass has been described above as the material of which the optical fibers are constructed. Any solid substance which is an optical conductor and to which polymers can be attached directly or by means of linking groups may be used instead of glass. Examples are plastics such as polymethylmethacrylate and polystyrene.

Suitable linking groups for glass and other fibers may be prepared from the following: diaminoalkanes, diaminoaryl compounds, diisothiocyanatesubstituted aryl compounds, alkyldialdehydes, arylalkyl dialdehydes, arylalkylediamines, alkyl dicarboxylic acid cerivatives, arylalkyl decarboxylic acid derivatives, aryl dicarboxylic acid derivates, alkyldiols, aryl diols, arylalkyl diols, or any appropriate organic compound capable of forming extramolecular bonds with at least two pendant chemical groups.

In the examples above fluorescent species such as compound <u>8</u> and FITC are used and the ratio of fluorescent light to reflected light is measured. As noted active species other than fluorescent species may be used. For example phenol red, cresol red and neutral red may be used. These dyes change color with pH. These dyes have functional groups which are capable of reacting with functional groups of monomers and polymers and can be incorporated in polymer molecules formed *in situ* or in

preformed polymers or they can be trapped in polymers as in Example 11. The following example will serve for purposes of illustration.

Example 15 - Incorporation of Cresol Red in a Polymer Chain

Cresol Red (0.038g) is dissolved in 30 ml of chloroform. N-Bromosuccinimide (0.017 g) is added along with benzoyl peroxide (0.005 g). The solution is refluxed for one hour and then allowed to cool to room temperature. The succinimide is removed by filtration and the bromomethyl cresol red is crystallized from acetic acid.

To a solution of dimethylformamide (25 ml) containing the bromomethyl cresol red derivative (0.025 g) is added a 10 fold molar excess of allylamine. The reaction mixture is heated with stirring at 50°C under argon in the dark for 6 hours. The reaction mixture is then cooled to room temperature and the solvent removed under high vacuum.

The reaction product of the bromomethyl cresol red and allylamine is dissolved in DMF (50 ml) together with 1 g of acrylamide and 10 mg 2,2'-azobisisobutyronitrile. Into this solution is then placed aminoalkyl glass, in the form of a fiber tip, which has been previously treated with a mixture of acryloyl chloride (0.10 g), pyridine (20 ml), and N,-N'-dimethylamino pyridine (0.010 g) for two hours. The resulting amidated fiber is heated with the bromomethyl cresol red derivative at 50° for 20 h. A copolymer of the bromomethyl cresol red allylamine product and acrylamide is formed at the end of the fiber.

A simplified reaction scheme is as follows:

Cresol red + N-bromosuccinimide $\xrightarrow{\Delta}$
Benzoyl peroxide

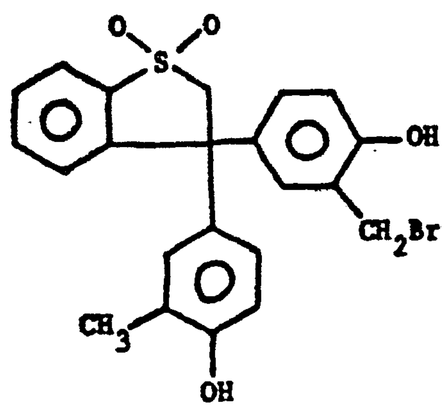

I

I + $NH_2-CH_2-CH=CH_2$ $\longrightarrow$

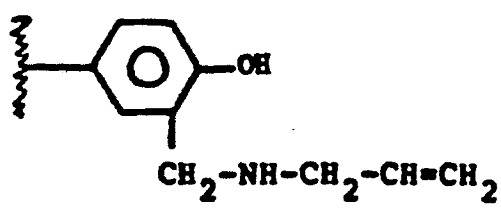

II

II + $CH_2=CH-CO-NH_2$ + $\vdash NH-CO-CH=CH_2$ $\longrightarrow$ III

In II, the remainder of the molecule is as in I. III is the glass fiber to which a copolymer of acrylamide and II is covalently attached.

It will be apparent that a new and useful method of making optical fibers having enhanced capacity for fluorescence, etc. and new and useful optical fibers having such enhanced properties have been provided.

CLAIMS:

1. An article comprising a solid substrate to a surface of which are attached a plurality of polymer molecules, the polymer molecules embodying a multiplicity of chemically, biochemically, or biologically active species.

2. An article as claimed in claim 1 wherein the solid substrate is an optical fiber and the polymer molecules are attached to one end of the fiber.

3. An article as claimed in claim 2 wherein the optical fiber is a glass fiber or an organic fiber.

4. An optical fiber as claimed in claim 2 or claim 3 in which the polymer molecules are attached covalently thereto.

5. An optical fiber as claimed in claim 4 in which the aforesaid species are fluorescent and are attached to the polymer by covalent bonds.

6. An optical fiber as claimed in claim 4 in which the aforesaid species are physically entrapped by the polymer molecules.

7. An optical fiber as claimed in claim 2 or claim 3 in which the polymer molecules are attached to the fiber by adsorption.

8. A method of preparing an optical fiber which is useful for sensing a chemical or physiological parameter of a body fluid or tissue

which comprises (a) providing an optical fiber having a distal end, and (b) treating the distal end of the fiber to attach to it a polymer embodying a multiplicity of molecular species which are capable of sensing the desired parameter.

9. A method as claimed in claim 9 wherein the polymer is attached covalently as a preformed polymer or by forming the polymer in situ.

10. A method as claimed in claim 8 wherein the polymer is attached by adsorption.

11. A method as claimed in any one of claims 8 to 10 which the aforesaid species are attached covalently to the polymer or to the monomer of oligomer from which it is formed.

12. A method as claimed in any one of claims 8 to 10 in which the aforesaid species are entrapped physically in the polymer.

European Patent Office

# EUROPEAN SEARCH REPORT

0205232
Application number

EP 86 30 2454

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | EP-A-0 072 627 (ICI)<br>* whole document * | 1-3,8 | G 01 N 21/64<br>G 01 N 21/77 |
| Y | | 4-6,9 | |
| | --- | | |
| Y | DE-A-3 343 636 (AVL AG)<br>* page 5, lines 5-9; page 6 * | 4-6,9 | |
| | --- | | |
| X | ANALYTICAL CHEMISTRY, vol. 56, no. 1, January 1984, pages 16A, 18A, 20A, 22A, 24A, 33A, 34A, Easton, Pennsylvania, US; W.R. SEITZ "Chemical sensors based on fiber optics" | 1-5,8 | |
| | --- | | TECHNICAL FIELDS SEARCHED (Int. Cl.4) |
| X | EP-A-0 109 959 (AVL AG)<br>* page 8, line 28 - page 9, line 9 * | 1 | |
| | --- | | G 01 N 21/00<br>G 01 D 5/26<br>A 61 B 5/00<br>G 01 N 33/00 |
| A | ANALYTICAL CHEMISTRY, vol. 56, no. 1, January 1984, pages 62-67, Easton, Pennsylvania, US; J.I. PETERSON et al.: "Fiber-optic probe for in vivo measurement of oxygen partial pressure" | 1-3 | |
| | --- | | |
| A,P | US-A-4 577 109 (T.B. HIRSCHFELD)<br>* whole document * | 1,8 | |
| | ----- | | |

The present search report has been drawn up for all claims

| Place of search | Date of completion of the search | Examiner |
|---|---|---|
| BERLIN | 04-07-1986 | BRISON O.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82